# FASCICULE DE BREVET EUROPEEN

(11) **EP 1 311 235 B1**
(45) Date de publication et mention de la délivrance du brevet: **03.01.2007**
(21) Numéro de dépôt: 01963098.7
(22) Date de dépôt: 14.08.2001
(51) Int. Cl.: A61K 8/891, A61K 8/892, A61Q 19/00

(54) **COMPOSITION COSMETIQUE ET/OU DERMATOLOGIQUE A ACTION HYDRATANTE AMELIOREE, COMPRENANT UN POLYSILOXANE RETICULE.**
KOSMETISCHE UND/ODER DERMATOLOGISCHE ZUSAMMENZETUNG MIT VERBESSERTER FEUCHTIGKEIT, ENTHALTEND EIN VERNETZENDES POLYSILOXAN
COSMETIC AND/OR DERMATOLOGICAL COMPOSITION WITH ENHANCED HYDRATING ACTIVITY, COMPRISING A CROSSLINKED POLYSILOXANE

(30) Priorité: 21.08.2000 FR 0010755
(43) Date de publication de la demande: 21.05.2003
(73) Titulaire: Laboratoire Nuxe, 75010 Paris (FR)
(72) Inventeur: LECLERE, Jacques, F-45500 SAINT-GONDON (FR)
(74) Mandataire: L'Helgoualch, Jean
(86) Numéro de dépôt international: PCT/FR2001/002613
(87) Numéro de publication internationale: WO 2002/015872

(56) Documents cités:
- EP-A- 0 908 175
- EP-A- 0 985 402
- EP-A- 1 093 805
- PATENT ABSTRACTS OF JAPAN vol. 1999, no. 11, 30 septembre 1999 (1999-09-30) & JP 11 158029 A (UNILEVER NV), 15 juin 1999 (1999-06-15)

## Description

La présente invention concerne l'utilisation d'un polymère réticulé à base de polysiloxane pour renforcer l'effet hydratant d'une composition cosmétique et/ou dermatologique comprenant un ou plusieurs agents hydratants. La peau comprend des couches superficielles, à savoir l'épiderme, et des couches plus profondes, le derme et l'hypoderme, et chacune possède des propriétés spécifiques permettant à l'ensemble de réagir et s'adapter aux conditions de son environnement. L'épiderme, qui constitue la couche externe, joue un rôle fondamental pour assurer la protection et le maintien d'une bonne trophicité. C'est pourquoi de nombreuses compositions ont été mises au point afin de le protéger et d'améliorer ses fonctions.

Ces compositions cosmétiques et dermatologiques destinées au traitement des affections de la peau par application topique doivent respecter certaines conditions pour être bien acceptées par les utilisateurs. Plus particulièrement, elles doivent posséder de bonnes propriétés physiques, notamment de consistance et d'onctuosité, présenter une efficacité satisfaisante, et être agréables à utiliser. De plus, elles doivent pouvoir être conservées dans des conditions normales de température et d'hygrométrie sans se dégrader sensiblement et en conservant leurs propriétés pendant des périodes prolongées.

De nombreux agents hydratants ont été utilisés dans les compositions cosmétiques, et par exemple des compositions sous forme d'émulsion ou de gel contenant de la glycérine (ou glycérol) sont décrites dans les brevets FR-A-2.742.354 et FR-A-2.703.907.

Les lécithines, ou phosphatidyl cholines, sont des phosphoglycérides généralement extraits de l'huile de soja. En raison de leurs propriétés rhéologiques, les lécithines sont souvent utilisées dans des émulsions pour conférer à la composition des propriétés de douceur et d'onctuosité. Ainsi par exemple, le brevet FR 2.777.179 décrit une composition cosmétique pour application topique comprenant une lécithine de soja en association avec de l'eau et un liquide hydrophobe tel qu'un hydrocarbure saturé ou une cyclométhicone, dans des proportions déterminées pour améliorer l'homogénéité des formulations. La demande WO 97.37637 décrit une composition à base de liposomes contenant une lécithine hydrogénée en combinaison avec un alcool (de préférence l'éthanol) destinée à faciliter la pénétration du principe actif (vitamine D) dans la peau.

On connaît divers composés du type silicone, c'est-à-dire des polymères synthétiques comportant des enchaînements Si-O, qui peuvent être réticulés pour former des élastomères. Les diméthicones sont des silicones du type polysiloxane qui peuvent être utilisés dans des compositions cosmétiques, notamment comme épaississants et comme matifiants.

On a constaté que l'effet hydratant d'une composition cosmétique ou dermatologique pouvait être potentialisé de manière surprenante par addition d'un polymère réticulé à base de silicone, et plus particulièrement de diméthicone.

La présente invention a donc pour objet l'utilisation d'un polymère réticulé à base de polysiloxane, et plus particulièrement à base de diméthicone, pour renforcer l'effet hydratant d'une composition cosmétique et/ou dermatologique comprenant un ou plusieurs agents hydratants

Outre les composants indiqués ci-dessus, la composition contient avantageusement une lécithine hydrogénée.

La lécithine hydrogénée utilisée dans l'invention est de préférence une lécithine de soja hydrogénée ou une lécithine de tournesol hydrogénée. Les lécithines disponibles dans le commerce contiennent généralement de la lécithine pure (phosphatidyl choline) en mélange avec d'autres phosphoglycérides tels que céphaline (en particulier la phosphatidyl éthanol-amine) et le phosphatidyl inositol. Conformément à l'invention on peut utiliser la lécithine pure hydrogénée ou une lécithine du commerce hydrogénée. Un exemple de lécithine hydrogénée utilisable dans l'invention est celle vendue dans le commerce sous la marque Emulmetik 320® par la société Lucas Mayer (lécithine de soja hydrogénée).

La lécithine hydrogénée peut en particulier être associée à un alcool à chaîne alkylée comportant 10 à 20 atomes de carbone, et notamment un alcool en C₁₂-C₁₆.

Suivant une forme préférentielle de réalisation, la composition comprend un agent hydratant ou humectant, en combinaison avec le polysiloxane réticulé, et en particulier le polymère réticulé de diméthicone.

L'agent hydratant ou humectant peut être choisi parmi un polyol, la glycérine (glycérol et des dérivés de glycérol), le sorbitol, le maltitol, le pentaérythritol, les polyacrylates et polyméthacrylates de glycéryle, les mucopolysaccharides tels que l'acide hyaluronique, des dérivés du chitosane et des dérivés de l'acide pyrrolidone carboxylique. D'une manière générale, tout agent hydratant convenant aux compositions cosmétiques peut être utilisé dans la présente invention. L'un des avantages procurés par la présente invention est donc de permettre de renforcer l'effet hydratant quel que soit l'agent hydratant utilisé, en le combinant au polysiloxane réticulé et en particulier au polymère réticulé de diméthicone, comme indiqué ci-dessus.

Le polymère réticulé peut être choisi de préférence parmi les polymères à haut poids moléculaire sous forme d'élastomère ou de poudre, et en particulier la cyclopentasiloxane / diméthicone, et la diméthicone / vinyl diméthicone ; ces composés sont disponibles dans le commerce par exemple sous les marques Dow Corning 9040 et Dow Corning 9506 respectivement.

Les quantités de polymère réticulé utilisées dans les compositions peuvent varier selon les effets recherchés et la nature des polymères. Dans le cas d'un polymère diméthicone / vinyl diméthicone, la proportion de polymère peut être comprise entre 0,5 et 5% en poids du poids total de la composition. Dans le cas d'un polymère siloxane / diméthicone, la quantité peut être plus élevée et la proportion peut être comprise entre 1 et 25% du poids total de la composition.

Il peut être avantageux d'incorporer dans la composition un polymère ou copolymère vinylique réticulé, et par exemple un copolymère à base d'acrylate de vinyle réticulé. Un tel additif améliore la stabilité de la composition et sa qualité de toucher.

La composition comprend donc en combinaison au moins un agent hydratant tel que le glycérol, et un polymère réticulé à base de polysiloxane, en association, le cas échéant avec une lécithine hydrogénée.

Les rapports en poids de ces composants sont de 0,1 % à 20 % pour l'agent hydratant, de 0,5 % à 25 % pour le polymère réticulé, et de 0,1 % à 8 % pour la lécithine hydrogénée.

Plus précisément, la teneur en lécithine hydrogénée est généralement comprise entre 0,1 et 8 % en poids par rapport au poids total de la composition, et de préférence entre 0,2 et 5 % en poids.

Les essais effectués avec des compositions ont montré que l'incorporation de la lécithine hydrogénée améliore sensiblement la stabilité de l'émulsion formée. Bien que les agents hydratants et la lécithine soient connus en cosmétique et puissent parfois être utilisés dans une même composition, rien ne permettait de prévoir que l'addition d'une lécithine hydrogénée apporterait une amélioration sensible aux propriétés de l'émulsion, en particulier sa stabilité.

La potentialisation de l'action hydratante a été observée par évaluation des moyennes d'hydratation au cours du temps entre des échantillons traités suivant l'invention et des échantillons non traités. On a constaté une augmentation du taux d'hydratation supérieure à 40% pour la composition par rapport à une composition identique mais ne contenant pas le polymère réticulé.

Les compositions sont destinées à être administrées par voie topique, par exemple par application ou pulvérisation sur la peau, et comprennent donc, outre les composants essentiels précités, divers excipients, additifs et supports usuels acceptables sur le plan dermatologique et cosmétique, choisis en fonction de leurs propriétés connues et de la forme galénique choisie. Ainsi, on peut incorporer dans la composition des conservateurs, des émulsionnants, des épaississants, des gélifiants hydrophiles ou lipophiles, des colorants, des antioxydants, des agents hydratants additionnels, des tensioactifs, des propulseurs pour aérosols, des parfums et divers additifs destinés à améliorer les propriétés physiques de la composition. Il peut aussi être avantageux d'incorporer dans la composition, des filtres ou écrans solaires choisis en fonction du degré de protection recherché.

Par exemple, on peut utiliser dans l'invention les conservateurs usuels de la technique des compositions dermatologiques ou cosmétologiques, et par exemple l'acide benzoïque ou un p-hydroxy-benzoate d'alkyle, et en particulier le p-hydroxy-benzoate de méthyle (Méthylparaben) et le p-hydroxy-benzoate de propyle (Propylparaben), isolément ou en combinaison.

Divers émulsionnants additionnels peuvent être choisis parmi ceux couramment employés dans la technique tels qu'un Polysorbate (par exemple le Tween 60® ou le Tween 80®), un ester de sorbitan tel que le stéarate ou le laurate de sorbitan, ou des dérivés d'acide stéarique comme le stéarate de PEG 100®, un stéarate de propylène glycol, un stéarate de polyéthylène glycol, un stéareth ou un cétéareth, ou encore un dérivé de sucrose (par exemple un ester) non éthoxylé. Suivant l'invention, l'émulsionnant additionnels est de préférence un dérivé de sucrose non éthoxylé.

On peut aussi ajouter des émollients tels qu'un malate d'alkyle, l'isohexadécane, des triglycérides d'acide caprique ou caprylique, etc.

Les gélifiants ou épaississants peuvent être choisis par exemple parmi les polyacrylamides (par exemple du type Carbopol), les copolymères acrylate/acide acrylique ou acrylamide/acide acrylamido propane sulfonique, les dérivés de cellulose comme l'hydroxypropyl cellulose, ou les gommes naturelles comme la gomme de xanthane.

Il peut aussi être avantageux d'incorporer dans la composition un actif complémentaire destiné à améliorer le comportement de la peau, tel que le tocophérol, la vitamine A (rétinol), l'acide rétinoïque, des agents bactéricides, etc.

Les compositions peuvent se présenter sous forme de crèmes, laits, émulsions huile-dans-eau (H/E) ou émulsions eau-dans-huile (E/H), lotions et émulsions pour pulvérisation, gels, masques ou pommades et de préférence sous forme de lotions ou émulsions sans alcool ou à faible teneur en alcool. Dans le cas de l'application par pulvérisation, les systèmes généralement utilisés sont soit par pompe atmosphérique, soit par poche souple ou par pression de gaz tel que air, azote ou dioxyde de carbone.

Les compositions peuvent être utilisées pour divers traitements cosmétologiques ou dermatologiques, en fonction des principes actifs qui y sont incorporés, par exemple pour la prévention et la diminution des rides, pour lutter contre le vieillissement de la peau lié à l'âge ou à l'exposition au soleil, ainsi que pour l'hydratation et la protection de la peau contre les diverses agressions de l'environnement, par exemple les agressions climatiques (vent, froid, pluie, etc.), chimiques (produits de nettoyage et détergents) ou encore la pollution.

Les exemples suivants illustrent l'invention plus en détail sans en limiter la portée. Sauf indication contraire, toutes les parties et pourcentages sont exprimés en poids.

### Exemple 1

On prépare une crème hydratante ayant la composition pondérale suivante :

| | | |
|---|---|---|
| Lécithine hydrogénée / Alcool C₁₂-C₁₆ / acide palmitique | | 4,00 |
| Cocoate de sucrose | | 1,00 |
| Trioctanoin | | 4,00 |
| Neopentanoate d'isodécyle | | 4,00 |
| Palmitate de cétyle | | 2,00 |
| Citrate tricaprylique | | 2,00 |
| Maléate dicaprylique | | 2,00 |
| Diméthicone / Vinyldiméthicone Crosspolymer (polymère de silicones réticulé) | | 6,00 |
| Diméthicone | | 1,00 |
| Huile de cacao | | 1,00 |
| Huile de Macadamia | | 1,50 |
| Tocophérol | | 0,70 |
| Palmitate de rétinyle | | 0,25 |
| Polyphénols de Pongamia | | 1,00 |
| Acide déhydroacétique | | 0,05 |
| Chlorphénésine | | 0,10 |
| Phénoxyéthanol | | 0,50 |
| EDTA tétrasodique | | 0,10 |
| Butylène glycol | | 5,00 |
| Méthyl- et Propyl-paraben | | 0,30 |
| Carbomer | | 0,20 |
| Protéines de soja | | 0,20 |
| Extrait de miel | | 5,00 |
| Lactate de méthyl silanol | | 5,00 |
| Trehalose | | 0,50 |
| Extrait d'algues | | 3,00 |
| Parfums | | 0,20 |
| Trisamino (trométhamine) | | 0,40 |
| Eau | q.s.p. | 100,00 |

L'émulsion dont la compositions est donnée ci-dessus peut être préparée par les techniques conventionnelles.

Par exemple, le dérivé de lécithine est tout d'abord hydraté dans un mélange d'eau, du glycol et des conservateurs ci-dessus (90% de l'eau utilisée) pendant environ 20 minutes à une température de 60 à 65°C, puis on ajoute les corps gras en fin d'hydratation sous agitation en en provoquant l'émulsification au moyen d'un agitateur rotatif à haute vitesse, en ajoutant les vitamines et les polyphénols de Pongamia en fin d'opération.

Une fois l'émulsion obtenue, on ajoute le Carbomer dispersé dans les 10% d'eau restants, puis on refroidit à environ 50°C et on neutralise en ajoutant la Trométhamine. Jusqu'à obtention de particules de taille inférieure à 150 nm.

Les extraits végétaux sont ajoutés lorsque la température et revenue à 40°C, puis les parfums.

### Exemple 2

Par une méthode usuelle de la technique, on prépare un sérum hydratant (gel) ayant la composition pondérale suivante.

| | | |
|---|---|---|
| Cyclopentasiloxane / diméthicone (polymère réticulé) | | 20,00 |
| Acrylate / C10-C30 Alkyl Acrylates Crosspolymer (polymère réticulé) | | 0,30 |
| Butylène glycol-1,3 | | 3,00 |
| EDTA trisodique | | 0,10 |
| Glycereth-7 | | 3,00 |
| Benzophénone-3 | | 0,50 |
| Tromethamine | | 0,40 |
| Phenoxyéthanol | | 0,50 |
| Méthyl- et Propyl-paraben | | 0,30 |
| Extrait de Sophora japonica | | 2,00 |
| Escine | | 0,50 |
| Protéines de soja | | 3,00 |
| Mucilages de baobab | | 5,00 |
| Mucilages de Tamarindus indica | | 4,00 |
| Extrait de Codium tomentosum | | 3,00 |
| Colorant | | 0,10 |
| Parfums | | 0,10 |
| Eau | q.s.p. | 100,00 |

On procède tout d'abord à la préparation d'un gel en mélangeant le polymère réticulé à base de cyclopentasiloxane avec de l'eau, puis on ajoute au gel formé l'acrylate, le glycol, l'EDTA et les conservateurs. Le mélange est neutralisé par addition de trométhamine et on ajoute la benzophénone et de l'eau (4 parties en poids), puis l'escine dissoute dans un peu d'eau. Les autres composants sont ensuite ajoutés progressivement sous agitation, en terminant par les parfums.

### Exemple 3

Par la même méthode que dans l'Exemple 2, on prépare un sérum hydratant ayant la même composition, mais en remplaçant le polymère réticulé cyclopentasiloxane / diméthicone par le copolymère réticulé (10%) diméthicone / vinyl diméthicone.

## Revendications

1. Utilisation d'un polymère réticulé à base de polysiloxane pour renforcer l'effet hydratant d'une composition cosmétique et/ou dermatologique comprenant un ou plusieurs agents hydratants.

2. Utilisation selon la revendication 1, **caractérisée en ce que** le polymère réticulé est à base de diméthicone.

3. Utilisation selon la revendication 2, **caractérisée en ce que** le polymère réticulé est choisi parmi la cyclopentasiloxane / diméthicone, et la diméthicone / vinyl diméthicone.

4. Utilisation selon la revendication 3, **caractérisée en ce que** la composition contient un polymère diméthicone / vinyl diméthicone en une teneur comprise entre 0,5 et 5% en poids du poids total de la composition.

5. Utilisation selon la revendication 3, **caractérisée en ce que** la composition contient un polymère cyclopentasiloxane / diméthicone en une teneur comprise entre 1 et 25% en poids du poids total de la composition.

6. Utilisation selon l'une quelconque des revendications précédentes, **caractérisée en ce que** la composition contient en outre une lécithine hydrogénée.

7. Utilisation selon la revendication 6, **caractérisée en ce que** la lécithine est une lécithine de soja hydrogénée ou une lécithine de tournesol hydrogénée.

8. Utilisation selon l'une quelconque des revendications précédentes, **caractérisée en ce que** la composition contient en outre un polymère ou copolymère vinylique réticulé.

9. Utilisation selon la revendication 1, **caractérisée en ce que** l'agent hydratant est choisi parmi la glycérine, le sorbitol, le maltitol, le pentaérythritol, les polyacrylates et polyméthacrylates de glycéryle, les mucopolysaccharides tels que l'acide hyaluronique, des dérivés du chitosane et des dérivés de l'acide pyrolidine carboxylique.

10. Utilisation selon l'une quelconque des revendications 1 et 9, **caractérisée en ce que** la teneur en agent hydratant est comprise entre 0,1 % et 20 %.

## Claims

1. The use of a crosslinked polymer based on polysiloxane in order to reinforce the moisturizing effect of a cosmetic and/or dermatologic composition comprising one or several moisturizing agents.

2. The use according to claim 1, **characterized in that** the crosslinked polymer is based on dimethicone.

3. The use according to claim 2, **characterized in that** the crosslinked polymer is selected from cyclopentasiloxane / dimethicone, and dimethicone / vinyl dimethicone.

4. The use according to claim 3, **characterized in that** the composition contains a dimethicone / vinyl dimethicone polymer with a content comprised between 0,5 and 5% by weight of the total weight of the composition.

5. The use according to claim 3, **characterized in that** the composition contains a cyclopentasiloxane / dimethicone polymer with a content comprised between 1 and 25% by weight of the total weight of the composition.

6. The use according to any one of the preceding claims, **characterized in that** the composition further contains a hydrogenated lecithin.

7. The use according to claim 6, **characterized in that** the lecithin is a hydrogenated soya lecithin or a hydrogenated sunflower lecithin.

8. The use according to any one of the preceding claims, **characterized in that** the composition further contains a crosslinked vinyl polymer or copolymer.

9. The use according to claim 1, characterissed in that the moisturizing agent is selected from glycerin, sorbitol, maltitol, pentaerythritol, glyceryl polyacrylates and polymethacrylates, mucopolysaccharides such as hyaluronic acid, chitosane derivatives and pyrolidine carboxylic acid derivatives.

10. The use according to any one of claims 1 and 9, **characterized in that** the content of moisturizing agent is comprised between 0,1 and 20%.

## Patentansprüche

1. Verwendung eines vernetzten Polymers auf Polysiloxanbasis zur Verstärkung der hydratisierenden Wirkung einer kosmetischen und/oder dermatologischen Zusammensetzung, die ein oder mehrere Hydratisierungsmittel umfasst.

2. Verwendung nach Anspruch 1, **dadurch gekennzeichnet, dass** das vernetzte Polymer auf Dimethicon basiert.

3. Verwendung nach Anspruch 2, **dadurch gekennzeichnet, dass** das vernetzte Polymer aus Cylcopentasiloxan/Dimethicon und Dimethicon/Vinyldimethicon ausgewählt ist.

4. Verwendung nach Anspruch 3, **dadurch gekennzeichnet, dass** die Zusammensetzung ein DimethiconNinyldimethicon-Polymer in einem Gehalt zwischen 0,5 und 5 Gew.-%, bezogen auf das Gesamtgewicht der Zusammensetzung, enthält.

5. Verwendung nach Anspruch 3, **dadurch gekennzeichnet, dass** die Zusammensetzung ein Cyclopentasiloxan/Dimethicon-Polymer in einem Gehalt zwischen 1 und 25 Gew.-%, bezogen auf das Gesamtgewicht der Zusammensetzung, enthält.

6. Verwendung nach einem der vorangegangenen Ansprüche, **dadurch gekennzeichnet, dass** die Zusammensetzung außerdem ein hydriertes Lecithin enthält.

7. Verwendung nach Anspruch 6, **dadurch gekennzeichnet, dass** das Lecithin ein hydriertes Sojalecithin oder ein hydriertes Sonnenblumenlecithin ist.

8. Verwendung nach einem der vorangegangenen Ansprüche, **dadurch gekennzeichnet, dass** die Zusammensetzung außerdem ein vernetztes Vinylpolymer oder -copolymer enthält.

9. Verwendung nach Anspruch 1, **dadurch gekennzeichnet, dass** das Hydratisierungsmittel aus Glycerin, Sorbit, Maltit, Pentaerythrit, Glycerylpolyacrylaten und -polymethacrylaten, Mucopolysacchariden, wie z.B. Hyaluronsäure, Derivaten von Chitosan und Derivaten von Hydroxyprolincarbonsäure ausgewählt ist.

10. Verwendung nach einem der Ansprüche 1 bis 9, **dadurch gekennzeichnet, dass** das der Gehalt des Hydratisierungsmittels zwischen 0,1 % und 20 % liegt.
